# EUROPEAN PATENT APPLICATION

(11) **EP 2 778 668 A1**
(43) Date of publication of application: **17.09.2014**
(21) Application number: 12846288.4
(22) Date of filing: 24.10.2012
(51) Int. Cl.: G01N 27/30, G01N 33/53, G01N 33/48

(54) **MULTI-REACTION BIOSENSOR**

(30) Priority: 31.10.2011 KR 20110111851
(71) Applicant: Ceragem Medisys Inc., Cheonan-si, Chungcheongnam-do 331-833 (KR)
(72) Inventor: LEE, Jin Woo, Cheonan-si Chungcheongnam-do 331-833 (KR); CHOI, Jae Kyu, Cheonan-si Chungcheongnam-do 331-833 (KR)
(74) Representative: Bridge, Kerry Ann
(86) International application number: PCT/KR2012/008786
(87) International publication number: WO 2013/065994

(57) **Abstract**

Provided is a multi-reaction biosensor capable of generating various kinds of reaction signals through introduction of a sample at a time.

The multi-reaction biosensor having a capillary flow path through which a sample is introduced, comprises a reaction substrate configured to form at least two wall surfaces of a plurality of wall surfaces that form the capillary flow path, and configured to generate and transmit a reaction signal according to a reaction with the introduced sample; and a base substrate coupled to the reaction substrate such that the capillary flow path has a polygonal cross-sectional shape, and configured to form a wall surface other than the wall surfaces formed by the reaction substrate. Accordingly, various kinds of reaction signal can be generated through introduction of a sample at a time.

## Description

### [Technical Field]

The present invention relates to a multi-reaction biosensor, and more particularly, to a multi-reaction biosensor capable of generating various kinds of reaction signals through introduction of a sample at a time.

### [Background Art]

Biosensors refer to means for investigating properties of a material using functions of a living organism, and has good sensitivity and reaction specificity because a biomaterial such as blood sugar, ketone, or the like, is used as a detection element. The biosensor is classified as an enzyme analysis method or an immunity analysis method according to an analysis type, and classified as an optical biosensor or an electrochemical biosensor according to a method of quantitatively analyzing an analysis target material in a living body sample. Such biosensors are used for various self tests and rapid disease diagnosis such as blood sugar measurement, pregnancy diagnosis, urine examination, and so on.

In the case of an electrochemical biosensor mainly used for blood sugar measurement, an electrical signal is generated by an electrochemical reaction caused when a sample such as blood is introduced into the biosensor to be transmitted to a measurement device connected or fastened to the biosensor.

Meanwhile, a biosensor capable of measuring various biomaterials on one substrate has been also developed. The biosensor configured to measure various biomaterials according to the related art has multi-reaction places on one substrate, and several reaction places are sequentially configured at one sample introduction path in a direction of a flowing sample.

In the biosensor of the related art, since the injected sample flows on the substrate and biomaterial reactions are performed in sequence of times when the sample arrives at electrodes of the reaction places, different biomaterials cannot be simultaneously reacted with one biosensor.

In addition, since the samples to be reacted at the reaction places should be introduced into the reaction places, the samples should be introduced several times, and an amount of sample should be abruptly increased to measure a plurality of biomaterials.

In addition, since the reaction places are sequentially configured on the single substrate in a sample introduction direction, the biomaterial reaction of a front reaction place in the sample introduction direction may exert an influence on the biomaterial reaction of a rear reaction place. That is, a biomaterial measurement value of the rear reaction place in the sample introduction direction may be influenced not to guarantee accuracy, reproducibility, or the like.

### [Summary of Invention]

### [Technical Problem]

The present invention has been devised in light of the above-mentioned circumstances, an object of the present invention is to provide a multi-reaction biosensor capable of generating a plurality of reaction signals through introduction of a sample at a time.

Another object of the present invention is to provide a multi-reaction biosensor capable of simultaneously measuring a plurality of same biomaterials or various different biomaterials using one biosensor.

Still another object of the present invention is to provide a multi-reaction biosensor capable of simultaneously measuring a plurality of same biomaterials or various different biomaterials through introduction of a sample at a time.

Still another object of the present invention is to provide a multi-reaction biosensor capable of measuring a plurality of same biomaterials or various different biomaterials through a simple structure having reaction substrates formed at surfaces thereof that constitute a path through which samples pass.

### [Solution to Problem]

In order to achieve the aforementioned objects, a multi-reaction biosensor of the present invention is a biosensor having a capillary flow path through which a sample is introduced, including a reaction substrate configured to form at least two wall surfaces of a plurality of wall surfaces that form the capillary flow path, and configured to generate and transmit a reaction signal according to a reaction with the introduced sample; and a base substrate coupled to the reaction substrate such that the capillary flow path has a polygonal cross-sectional shape, and configured to form a wall surface other than the wall surfaces formed by the reaction substrate.

In addition, more preferably, the reaction substrate may be constituted by at least one upper reaction substrate that forms an upper wall surface, and at least one lower reaction substrate that forms a lower wall surface opposite to the upper wall surface.

Further, more preferably, the biosensor may further include comprising an intermediate reaction substrate disposed at predetermined intervals between the reaction substrates that form the upper wall surface and the lower wall surface, having both side surfaces fixed to the base substrate, and configured to generate and transmit a reaction signal according to a reaction with the introduced sample.

Furthermore, more preferably, the reaction substrate may be constituted by at least one upper reaction substrate that forms an upper wall surface, and at least one side reaction substrate that forms a side wall surface in contact with the upper wall surface.

In addition, more preferably, the capillary flow path may have any one cross-sectional shape of a triangular shape, a rectangular shape, a pentagonal shape, and a hexagonal shape.

Further, more preferably, the base substrate is provided with at least one guide member to which the reaction substrate is coupled may be installed.

Furthermore, more preferably, the base substrate is provided with an insertion rail recessed to a predetermined depth into which the reaction substrate is coupled may be installed.

In addition, more preferably, at least one of the base substrate and the reaction substrate is provided with an air discharge section configured to be penetrated such that air in the capillary flow path is discharged may be installed.

Further, more preferably, the reaction substrate may include an electrode section configured to react with a target biomaterial to generate a reaction signal; and a signal transmission unit configured to transmit the reaction signal to a measurement device, and the electrode section is constituted by a reaction electrode and a reference electrode to generate a reaction signal.

In order to accomplish the above-mentioned objects, a multi-reaction biosensor of the present invention is a biosensor having a capillary flow path through which a sample is introduced, including: at least two reaction substrates configured to form at least one wall surface of a plurality of wall surfaces that form the capillary flow path, and generate and transmit a reaction signal according to a reaction with the introduced sample; and a base substrate coupled to the reaction substrate such that the capillary flow path has a polygonal cross-sectional shape, and configured to form a wall surface other than the wall surface formed by the at least two reaction substrates, wherein the reaction substrate is constituted by an electrode section reacted with a target biomaterial to generate a reaction signal; and a signal transmission unit configured to transmit the reaction signal to a measurement device.

### [Advantageous Effects of Invention]

As described above, the multi-reaction biosensor according to the present invention can simultaneously cause reactions of different biomaterials using one biosensor, and the plurality of same biomaterials or various different biomaterials can be measured, improving workability.

In addition, according to the present invention, the plurality of biomaterials can be simultaneously reacted through mere introduction of the sample at a time, and the plurality of different biomaterials can be simultaneously detected using the same amount of sample without increasing the amount of sample.

Further, according to the present invention, biomaterial reaction signal compensation can be easily performed by differentiating configurations and loadings of reagents (enzymes) on the plurality of reaction substrates to improve performance such as accuracy, reproducibility, or the like, of measurement values. Furthermore, the simultaneous reactions exert no influence on the biomaterial reactions on the reaction substrate to improve performance such as accuracy, reproducibility, or the like, of measurement values.

### [Brief Description of Drawings]

FIG. 1 is a perspective view showing a multi-reaction biosensor according to a first exemplary embodiment of the present invention;
FIG. 2 is an exploded perspective view of the multi-reaction biosensor shown in FIG. 1;
FIG. 3 is a cross-sectional view of the multi-reaction biosensor shown in FIG. 1;
FIG. 4 is a cross-sectional view showing a multi-reaction biosensor according to a second exemplary embodiment of the present invention;
FIG. 5 is a cross-sectional view showing a multi-reaction biosensor according to a third exemplary embodiment of the present invention;
FIG. 6 is a cross-sectional view showing a multi-reaction biosensor according to a fourth exemplary embodiment of the present invention;
FIG. 7 is a cross-sectional view showing a multi-reaction biosensor according to a fifth exemplary embodiment of the present invention;
FIG. 8 is a cross-sectional view showing a multi-reaction biosensor according to a sixth exemplary embodiment of the present invention;
FIG. 9 is a cross-sectional view showing a multi-reaction biosensor according to a seventh exemplary embodiment of the present invention;
FIGS. 10A and 10B are plan views showing structures of reaction substrates according to the embodiments of the present invention; and
FIGS. 11A to 11C are views showing coupling states of the reaction substrates according to the embodiments of the present invention.

### [Description of Embodiments]

Hereinafter, a multi-reaction biosensor according to an exemplary embodiment of the present invention will be described in detail with reference to the accompanying drawings.

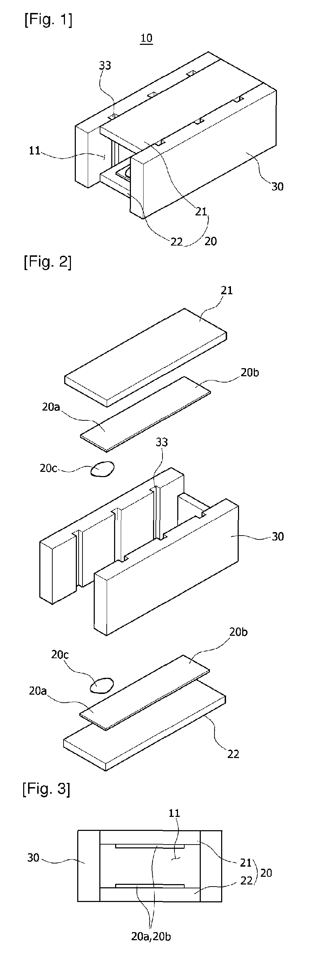

FIG. 1 is a perspective view showing a multi-reaction biosensor according to a first exemplary embodiment of the present invention, FIG. 2 is an exploded perspective view of the multi-reaction biosensor shown in FIG. 1, and FIG. 3 is a cross-sectional view of the multi-reaction biosensor shown in FIG. 1.

As shown in FIGS. 1 to 3, a multi-reaction biosensor 10 according to the first exemplary embodiment of the present invention has a capillary flow path 11 formed of a hexahedral pipe shape and passing through a center thereof. While a cross-sectional shape of the capillary flow path 11 is exemplarily described as being a rectangular cross-sectional shape, the cross-sectional shape is not limited thereto but may be configured as various polygonal cross-sectional shapes such as a triangular, pentagonal, hexagonal shape, or the like, as well as the rectangular cross-sectional shape, according to a user's purpose. The present invention includes all of the capillary flow path 11 having the polygonal cross-sectional shapes.

An upper wall surface and a lower wall surface of the biosensor 10 are formed of a reaction substrate 20, and side wall surfaces are formed of base substrates 30. The upper wall surface is formed of an upper reaction substrate 21, and the lower wall surface opposite to the upper wall surface is formed of a lower reaction substrate 22.

The base substrates 30 form the side wall surfaces that maintain a constant interval. The base substrates 30 are connected to each other by an intermediate member, coupled to the reaction substrates 20 to form the capillary flow path 11, and have one ends configured as an opening section and the other ends coupled to the reaction substrates 20 to form a closed structure. An air discharge section 33 configured to be penetrated and in communication with the outside so that air in the capillary flow path 11 is discharged is formed at the biosensor 10 to which the base substrates 30 and the reaction substrates 20 are coupled. As shown, the air discharge section 33 may be recessed in the inner side surface of the base substrate 30 to a predetermined depth and may extend to an end thereof in a vertical direction. However, the air discharge section 33 is not limited to only the shown shape but may be formed at any position such as an intermediate section of the base substrate 30 or a periphery section of the reaction substrate 20 as long as the air discharge section 33 has a hole shape configured to bring the capillary flow path 11 in communication with the outside.

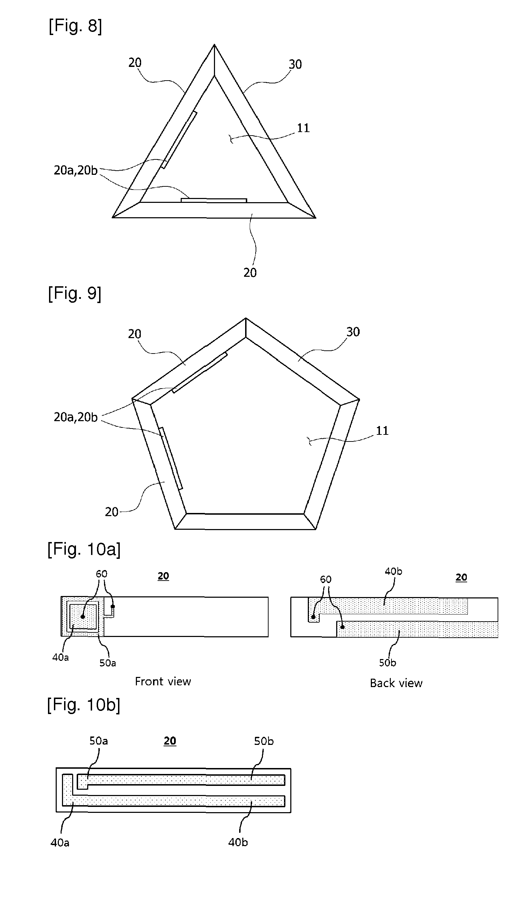

The reaction substrate 20 may be formed of a printed circuit board (PCB) substrate, or a flexible PCB (FPCB) substrate, and an electrode section 20a configured to react with a target biomaterial to generate a reaction signal and a signal transmission unit 20b configured to transmit the reaction signal to a measurement device are installed on one surface of the reaction substrate 20. Specifically, the electrode section 20a is constituted by an operating electrode and a reference electrode, and the signal transmission unit 20b is constituted by an operation signal transmitting electrode electrically connected to the operating electrode and a reference signal transmitting electrode electrically connected to the reference electrode. FIGS. 10A and 10B shows a structure of the reaction substrate 10. As shown in FIG. 10A, the reaction substrate 20 may have an operating electrode 40a and a reference electrode 50a formed at one surface, and an operation signal transmitting electrode 40b electrically connected to the operating electrode 40a and a reference signal transmitting electrode 50b electrically connected to the reference electrode 50a, which are formed on the other surface. Here, the operating electrode 40a may have a rectangular shape, and the reference electrode 50a may have a hollow rectangular shape surrounding the operating electrode 40a. The operating electrode 40a and the operation signal transmitting electrode 50a, and the reference electrode 50b and the reference signal transmitting electrode 50b may be electrically connected through a via-hole 60 passing through the reaction substrate 20. Alternatively, as shown in FIG. 10B, all of the operating electrode 40a and the reference electrode 50a, the operation signal transmitting electrode 40b electrically connected to the operating electrode 40a, and the reference signal transmitting electrode 50b electrically connected to the reference electrode 50a may be formed on the same surface of the reaction substrate 20. Shapes of the electrodes 40a, 40b, 50a and 50b may be variously deformed as long as the chemical reaction (or electrochemical reaction) and reaction signal transmission are not interfered in corresponding regions.

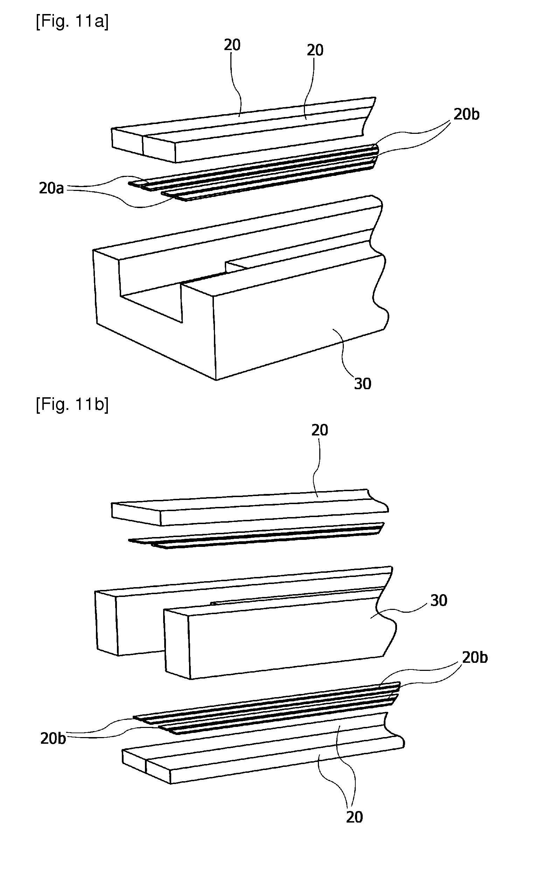

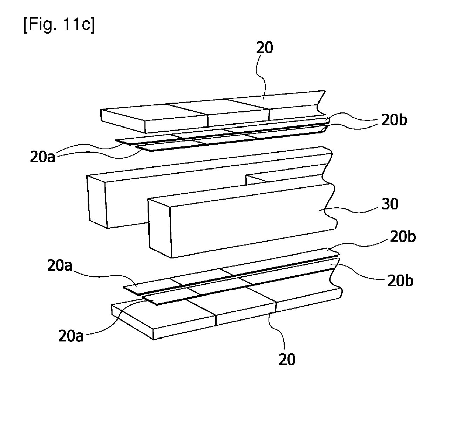

A reagent 20c is applied on an upper surface of the electrode section 20a. At least one operating electrode and at least one reference electrode are formed at facing surfaces of the reaction substrate 20, and have a facing electrode structure to form the signal transmission unit 20b formed of a conductive wire. The reagent 20c configured to cause an expected electrochemical reaction with a biomaterial serving as a measurement target is placed on a portion of the electrode section 20a. At least one of different reagents 20c may be placed on each of the electrode sections 20a. The reagent 20c causes a chemical reaction such as an oxidation-reduction reaction with the measurement target biomaterial, and is applied on the reaction electrode and fixed through a dry method or the like. In addition, while not shown, the plurality of pairs of electrode sections 20a and signal transmission units 20b may be formed at the inner side surface of the reaction substrates 20, in addition to the configuration having the pair of electrode sections 20a and signal transmission units 20b. Alternatively, at least two reaction substrates 20 may be installed at upper and lower portions of the base substrate 30. FIGS. 11A to 11C are views showing coupling shapes of the reaction substrates according to the embodiments of the present invention. As shown in FIG. 11A, two reaction substrates 20 may be coupled to only the upper portion of the base substrate 30. Alternatively, as shown in FIG. 11B, one reaction substrate 20 may be coupled to the upper portion of the base substrate 30, and two reaction substrates 20 may be coupled to the lower portion. Alternatively, as shown in FIG. 11C, the plurality of reaction substrates 20 may be coupled to the upper and lower portions of the base substrate 30. As described above, the coupling shape of the plurality of reaction substrates 20 is not limited to FIGS. 11A to 11C but various coupling shapes may be made as long as a basic frame is not interfered.

The base substrate 30 may be formed of a synthetic resin material and integrally formed through a processing method such as injection molding or the like, so that various structures may be provided. In addition, since a separate spacer, i.e., a blood supply layer, is not needed, a manufacturing process can be simplified.

While not shown, a means configured to detect sample introduction or a means configured to provide sensor identification information may be further installed at the base substrate 30 or the reaction substrate 20.

A biomaterial reaction may need a very small amount of sample 20c, and another biomaterial reaction may need a relatively large amount of sample 20c. For this, a reaction chamber space of the base substrate 30 can be adjusted. That is, a height between the reaction substrates 20 can be adjusted. In addition, the reaction electrode having a relatively large area may be needed for biomaterial reaction signal amplification. For this, an area of the corresponding reaction electrode may be adjusted in consideration of a signal amplification magnitude.

In the exemplary biosensor for multiple reaction of the present invention having the above-mentioned configuration, the base substrate 30 and the reaction substrate 20 are coupled to form a sample introduction port and a reaction chamber. The reaction chamber is a space in which an expected electrochemical reaction is generated with respect to a measurement target biomaterial (blood sugar, ketone, or the like). An opening of the space functions as a sample introduction port. When the sample (blood, saliva, urine, or the like) comes in contact with the sample introduction port, the sample is rapidly suctioned into the reaction chamber by a capillary tube phenomenon. The reagents 20c fixed onto the upper and lower electrode sections 20a in the reaction chamber meet with the corresponding measurement target biomaterial of the sample through suction of the sample to cause the electrochemical reaction and generate a reaction signal. The reaction signal is transmitted to a measurement device through the signal transmission unit 20b connected to the reaction electrode, and the measurement device calculates a measurement value based on the transmitted reaction signal.

The multi-reaction biosensor of the present invention can simultaneously detect a plurality of different biomaterial reaction signals using the same amount of sample without increasing the amount of sample. For example, as the one sample (blood) is merely introduced at a time, biomaterials such as blood sugar, total cholesterol, LDL cholesterol, HDL cholesterol, TG (triglyoerides), hemoglobin, ketone, uric acid, glycosylated hemoglobin (HbAlc), and so on, can be simultaneously reacted to obtain measurement values of the biomaterials. For example, even when a small amount of blood is simply introduced, since a blood sugar measurement reagent applied on the electrode section 20a of the lower reaction substrate 22 reacts with blood sugar of the blood and a ketone measurement reagent applied on the electrode section 20a of the upper reaction substrate 21 reacts with ketone of the blood, two kinds of biomaterials can simultaneously react to measure the values. Here, the biomaterial may be a biomaterial, which may be measured for clinical diagnosis, biomaterial measurement value compensation, or the like. Since the biosensor can be structurally and easily expanded and modified, various modifications may be made. For example, a specified enzyme is placed on a reaction substrate 20 and another specified enzyme is placed on another reaction substrate 20, and the biomaterial reaction signal detected at the other the reaction substrate 20 may be compensated by the signal value detected at the one reaction substrate 20 to improve performance such as accuracy, reproducibility, or the like, of the biomaterial measurement value. For another example, the reagent including the specified enzyme is placed on the reaction substrate 20 and the reagent is not placed on the other reaction substrate 20, and thus, noise and interference of the biosensor 10 can be removed using a background signal detected on the reaction substrate 20 with no reagent.

The sample introduced into the single sample introduction port flows in different sample introduction directions, i.e., directions of the upper reaction substrate 21 and the lower reaction substrate 22 through the reaction chamber space. In the biosensor of the related art, while the biomaterial reaction of the front reaction place exerts an influence on the biomaterial reaction of the rear reaction place, since the biomaterial reaction on the reaction substrate 20 of the biosensor of the present invention exerts no influence on the biomaterial reaction of the other reaction substrate 20, performance such as accuracy, reproducibility, or the like, of the measurement value can be improved.

Exemplarily describing the reagent placed on the reaction substrate 20, a reagent (buffer, polymer, surfactant, mediator, stabilizer, glucose-oxidized enzyme (GDH, GOD)) reacted with the blood sugar (for measuring blood sugar) is placed on the upper reaction substrate 21 coupled to the upper portion of the base substrate 30, and a reagent reacted with cholesterol (for measuring cholesterol) is placed on the lower reaction substrate 22 coupled to the lower surface of the base substrate 30. A blood sugar reaction signal is detected on the upper reaction substrate 21, and a cholesterol reaction signal is detected on the lower reaction substrate 22. The blood sugar reaction signal and the cholesterol reaction signal are transmitted to the measurement device via the signal transmission unit 20b of the reaction substrate 20, and the measurement device obtains a measurement vale based on the corresponding biomaterial reaction signal.

Describing another example, a reagent for measuring blood sugar is placed on the upper reaction substrate 21 coupled to the upper portion of the base substrate 30, and a reagent (buffer, polymer, surfactant, hemoclastic, mediator, stabilizer) for measuring hemoglobin is placed on the lower reaction substrate 22 coupled to the lower portion of the base substrate 30. The blood sugar reaction signal is detected on the upper reaction substrate 21, and the hemoglobin reaction signal is detected on the lower reaction substrate 22. The blood sugar reaction signal and the hemoglobin reaction signal are transmitted to the measurement device via the signal transmission unit 20b of the reaction substrate 20, and the measurement device compensates the blood sugar reaction signal detected on the upper reaction substrate 21 with the hemoglobin reaction signal detected on the lower reaction substrate 22 to obtain a correct blood sugar measurement value, from which an influence of an hematocrit (HCT) effect is removed.

As described above, in the biosensor 10, the blood sugar reaction of the upper reaction substrate 21 exerts no influence on the hemoglobin reaction of the lower reaction substrate 22, and similarly, the hemoglobin reaction of the lower reaction substrate 22 exerts no influence on the blood reaction of the upper reaction substrate 21. That is, since the electrode section 20a of the upper reaction substrate 21 is separated from the electrode section 20a of the lower reaction substrate 22, hematocrit compensation can be accurately performed.

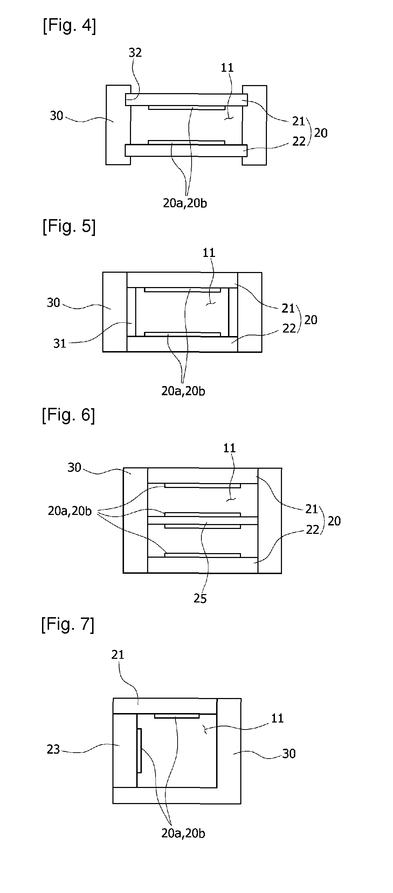

Similarly, as shown in FIG. 4, in a multi-reaction biosensor according to a second exemplary embodiment of the present invention, an insertion rail 32 recessed to a predetermined depth and extending in a longitudinal direction is formed at an inner side surface of the base substrate 30 in contact with the capillary flow path 11. The insertion rails 32 are formed at the facing inner side surfaces of the base substrate 30 to form a pair. The insertion rails 32 may be formed at the upper end section and the lower end section of the base substrate 30. Both side surfaces of the upper reaction substrate 21 and the lower reaction substrate 22 are inserted and fixed into the insertion rails 32. Here, as described above, a vertical interval of the insertion rails 32 can be adjusted to adjust an internal space size of the capillary flow path 11. While not shown, the insertion rails 32 may be formed at equal intervals and an interval between the upper reaction substrate 21 and the lower reaction substrate 22 may be adjusted according to the kind of measurement operations. In addition, while not shown, the plurality of pairs of the electrode sections 20a and the signal transmission units 20b may be formed at the inner side surface of the reaction substrate 20, in addition to the configuration having the pair of electrode sections 20a and signal transmission units 20b. In addition, as described above, two reaction substrates 20 may be installed at least one of the upper end section and the lower end section of the base substrate 30.

A structure and a measurement process are similar to the first exemplary embodiment of the present invention except for a structure in which the insertion rail 32 is formed at the base substrate 30 to fix the upper reaction substrate 21 and the lower reaction substrate 22.

Similarly, as shown in FIG. 5, a multi-reaction biosensor according to a third exemplary embodiment of the present invention includes guide members 31 formed at the inner side surfaces of the base substrates 30 in contact with the capillary flow path 11, protruding to a predetermined height and extending in the longitudinal direction. The guide members 31 are formed at the facing inner side surfaces of the base substrates 30 to form a pair. Here, as described above, a width of the guide member 31 may be adjusted to adjust an internal space size of the capillary flow path 11. While not shown, the plurality of guide members 31 may be formed at equal intervals to adjust an interval between the upper reaction substrate 21 and the lower reaction substrate 22 according to the kind of measurement operations. In addition, while not shown, the pair of electrode sections 20a and signal transmission units 20b may be formed at the inner side surfaces of the reaction substrates 20 to form a plurality of pairs.

A structure and a measurement process are similar to the first exemplar embodiment of the present invention except for a structure in which the guide member 31 is formed on the base substrate 30 to fix the upper reaction substrate 21 and the lower reaction substrate 22.

Similarly, as shown in FIG. 6, a multi-reaction biosensor according to a fourth exemplary embodiment of the present invention includes an intermediate reaction substrate 25 mounted between the upper reaction substrate 21 that forms the upper wall surface and the lower reaction substrate 22 that forms the lower wall surface at an equal interval. Both side surfaces of the intermediate reaction substrate 25 are fixed to the inner side surfaces of the base substrates 30. The electrode section 20a and the signal transmission unit 20b are formed at one side surface or both side surfaces of the intermediate reaction substrate 25. The plurality of intermediate reaction substrates 25 may be mounted. Here, the interval between the intermediate reaction substrates 25 can be adjusted according to the kind of measurement operation. In addition, while not shown, the electrode sections 20a and the signal transmission units 20b may be formed at the inner side surfaces of the reaction substrates 20 to form a plurality of pairs, in addition to the structure in which the pair of electrode sections 20a and the pair of signal transmission units 20b are formed.

That is, the single reaction chamber space may be formed as a multi-reaction chamber space. The reaction chamber spaces formed by the upper reaction substrate 21 and the lower reaction substrate 22 of the sample introduction port side of the base substrate 30 are defined by the intermediate reaction substrate 25 configured to horizontally block the space in a vertical direction. The sample flows from the sample introduction port to a space of the reaction chamber space near the upper end of the intermediate reaction substrate 25 to be introduced into between the upper reaction substrate 21 and the intermediate reaction substrate 25. In addition, the sample flows from the sample introduction port to a space of the reaction chamber space near the lower end of the intermediate reaction substrate 25 to be introduced into between the lower reaction substrate 22 and the intermediate reaction substrate 25.

As described above, in the biosensor having the multi-reaction chamber space, a height of forming the intermediate reaction substrate 25 can be adjusted to equalize the size of the reaction chamber space near the upper reaction substrate 21 and the size of the reaction chamber space of the lower reaction substrate 22, or any one of both of the reaction chamber spaces may be increased for the case in which any one of both of the reaction chamber spaces requires a larger amount of sample. In addition, for the case in which the biomaterial reactions of the upper reaction chamber space and the lower reaction chamber space are affected by the sample, in a structure in which a portion of the entire shaft in the horizontal direction of the intermediate reaction substrate 25 is formed and the remaining portion is open, the upper reaction chamber space and the lower reaction chamber space may adjust the reaction chamber space formed by the intermediate reaction substrate 25 to adjust an amount of sample needed for the reaction substrates 20 (the upper reaction substrate, the lower reaction substrate and the intermediate reaction substrate).

A structure and a measurement process are similar to the first exemplary embodiment of the present invention except for the structure in which the intermediate reaction substrate 25 is mounted on the base substrate 30.

Similarly, as shown in FIG. 7, a multi-reaction biosensor of a fifth exemplary embodiment of the present invention is constituted by the upper reaction substrate 21 that forms the upper wall surface, a side reaction substrate 23 that forms a side wall surface in contact with the upper wall surface, and the base substrate 30 that forms a side wall surface and a lower wall surface opposite to the side wall surface at which the side reaction substrate 23 is formed.

Similar to the upper reaction substrate 21, the electrode section 20a and the signal transmission unit 20b are also formed at the side reaction substrate 23. In addition, the electrode section 20a and the signal transmission unit 20b may also be formed at still another side wall surface and lower wall surface. In addition, while not shown, the electrode sections 20a and the signal transmission units 20b may be formed at the inner side surfaces of the reaction substrates 20 to form a plurality of pairs, in addition to the structure in which the pair of electrode sections 20a and the pair of signal transmission units 20b are formed.

A configuration and a measurement process are similar to the first exemplary embodiment of the present invention except for the structure in which the side reaction substrate 23 in contact with the upper reaction substrate 21 is mounted.

Similarly, as shown in FIG. 8, a multi-reaction biosensor of a sixth exemplary embodiment of the present invention includes the capillary flow path 11 having a triangular cross-sectional shape. The capillary flow path 11 is constituted by the reaction substrates 20 mounted on at least two side surfaces and the base substrate 30 forming the remaining side surface to form a triangular cross-sectional shape. The electrode section 20a and the signal transmission unit 20b are formed at the reaction substrate 20. In addition, similarly, as shown in FIG. 9, a multi-reaction biosensor of a seventh exemplary embodiment of the present invention includes the capillary flow path 11 having a pentagonal cross-sectional shape. The reaction substrates 20 mounted on at least two side surfaces, and the base substrates 30 that form the remaining side surfaces are configured to form a polygonal cross-sectional shape. The electrode section 20a and the signal transmission unit 20b are formed at the reaction substrate 20. In addition, while not shown, a plurality of polygonal shapes may be formed in addition to the above-mentioned triangular, rectangular, pentagonal, and hexagonal shapes.

The multi-reaction biosensors according to the sixth and seventh exemplary embodiments of the present invention have the same structure and operating process as the first exemplary embodiment of the present invention except for the structure in which the reaction substrate 20 and the base substrate 30 are configured to form the triangular and pentagonal cross-sectional shapes of the capillary flow path 11.

While the invention has been shown and described with reference to certain example embodiments thereof, it will be understood by those skilled in the art that various changes in form and details may be made therein without departing from the spirit and scope of the invention as defined by the appended claims.

## Claims

1. A multi-reaction biosensor (10) having a capillary flow path (11) through which a sample is introduced, the biosensor (10) comprising:
a reaction substrate (20) configured to form at least two wall surfaces of a plurality of wall surfaces that form the capillary flow path (11), and configured to generate and transmit a reaction signal according to a reaction with the introduced sample; and
a base substrate (30) coupled to the reaction substrate (20) such that the capillary flow path (11) has a polygonal cross-sectional shape, and configured to form a wall surface other than the wall surfaces formed by the reaction substrate (20).

2. The multi-reaction biosensor according to claim 1, wherein the reaction substrate (20) is constituted by at least one upper reaction substrate (21) that forms an upper wall surface, and at least one lower reaction substrate (22) that forms a lower wall surface opposite to the upper wall surface.

3. The multi-reaction biosensor according to claim 1, further comprising an intermediate reaction substrate (25) disposed at a constant interval between the reaction substrates (20) that form the upper wall surface and the lower wall surface, having both side surfaces fixed to the base substrate (30), and configured to generate and transmit a reaction signal according to a reaction with the introduced sample.

4. The multi-reaction biosensor according to claim 1, wherein the reaction substrate (20) is constituted by at least one upper reaction substrate (21) that forms an upper wall surface, and at least one side reaction substrate (23) that forms a side wall surface in contact with the upper wall surface.

5. The multi-reaction biosensor according to claim 1, wherein the capillary flow path (11) has any one cross-sectional shape of a triangular shape, a rectangular shape, a pentagonal shape, and a hexagonal shape.

6. The multi-reaction biosensor according to claim 1, wherein the base substrate (30) is provided with at least one guide member (31) to which the reaction substrate (20) is coupled.

7. The multi-reaction biosensor according to claim 1, wherein the base substrate (30) is provided with an insertion rail (32) recessed to a predetermined depth into which the reaction substrate (20) is coupled is installed.

8. The multi-reaction biosensor according to any one of claims 1 to 7, wherein at least one of the base substrate (30) and the reaction substrate (20) is provided with an air discharge section (33) configured to be penetrated such that air in the capillary flow path (11) is discharged is installed.

9. The multi-reaction biosensor according to any one of claims 1 to 7, wherein the reaction substrate (20) comprises:
an electrode section configured to react with a target biomaterial to generate a reaction signal; and
a signal transmission unit configured to transmit the reaction signal to a measurement device, and
wherein the electrode section is constituted by a reaction electrode and a reference electrode to generate a reaction signal.

10. A multi-reaction biosensor (10) having a capillary flow path (11) through which a sample is introduced, the biosensor (10) comprising:
at least two reaction substrates (20) configured to form at least one wall surface of a plurality of wall surfaces that form the capillary flow path (11), and generate and transmit a reaction signal according to a reaction with the introduced sample; and
a base substrate (30) coupled to the reaction substrate (20) such that the capillary flow path (11) has a polygonal cross-sectional shape, and configured to form a wall surface other than the wall surface formed by the at least two reaction substrates (20),
wherein the reaction substrate (20) is constituted by an electrode section reacted with a target biomaterial to generate a reaction signal; and
a signal transmission unit configured to transmit the reaction signal to a measurement device.
